Europäisches Patentamt

European Patent Office

Office européen des brevets

(11) Publication number: **0 074 248**
**B1**

(12) **EUROPEAN PATENT SPECIFICATION**

(45) Date of publication of patent specification: **19.02.86**

(51) Int. Cl.⁴: **A 61 K 39/00**

(21) Application number: **82304601.6**

(22) Date of filing: **01.09.82**

(54) Vaccine for hepatitis.

(30) Priority: **04.09.81 US 299452**

(43) Date of publication of application:
**16.03.83 Bulletin 83/11**

(45) Publication of the grant of the patent:
**19.02.86 Bulletin 86/08**

(84) Designated Contracting States:
**AT BE CH DE FR GB LI NL SE**

(56) References cited:
**US-A-4 046 871**

**CHEMICAL ABSTRACTS, vol. 95, no. 23,
December 7, 1981, page 499, abstract no.
201845q, COLUMBUS OHIO (US)**

**CHEMICAL ABSTRACTS, vol. 95, no. 23,
December 7, 1981, page 499, abstract no.
201846r, COLUMBUS OHIO (US)**

**CHEMICAL ABSTRACTS, vol. 90, no. 25, June
18, 1979, page 459, abstract no. 202079b,
COLUMBUS OHIO (US)**

(73) Proprietor: **THE REGENTS OF THE UNIVERSITY
OF CALIFORNIA**
**2490 Channing Way**
**Berkeley California 94720 (US)**

(72) Inventor: **Vyas, Girish Narmadashankara**
**79 Muth Drive**
**Orinda California (US)**

(74) Representative: **Harrison, David Christopher
et al**
**MEWBURN ELLIS & CO 2/3 Cursitor Street
London EC4A 1BQ (GB)**

Courier Press, Leamington Spa, England.

### Description

Hepatitis is a viral disease which can exist in both infectious and non-infectious form. It is of particular concern because of its transmission through blood transfusions. It is known that there are many carriers of the virus which are not subject to the symptoms of the disease. Nevertheless, these people are able to transmit the disease to others who are susceptible to infection, particularly through blood transfusions, where the blood is not carefully monitored.

Because of the widespread character of hepatitis, it would be desirable to be able to vaccinate people for the disease. For the most part, vaccines have relied upon the protein coat of the virus, which is only difficultly attainable and must be carefully purified to avoid any inclusion of the viral chromosome. Because of the expensive nature of the isolation and purification of the protein coat, an inexpensive vaccine has not been available which could be used, particularly in those areas which are unable to afford the high cost of the presently available vaccines.

Human serum albumin and the hepatitis B surface antigen are associated with the viral coat protein of the hepatitis B virus. Neurath et al. (1974) PNAS USA *71*:2663; Ionescu-Matiu et al. (1980) J. Med. Virol. *6*:175; Tiollais et al. (1981) Science *213*:406. Thus, common occurrence of albumin molecules in the coat protein of viruses replicating in the liver is naturally expected. *In vitro* aging or heat or glutaraldehyde polymerization of albumin results in a product which elicits antibodies in immunized animals. Onica et al. (1980) Mol. Immunol. *17*, 783. Antibodies to polymerized albumin have also been encountered in the sera of patients with acute or chronic liver disease. Lenkei et al. (1977) J. Med. Virol. *1*, 29. Physiological and pathological production of antibodies to polymerized human albumin (PHALB) have been studied by Onica and Lenkei (Onica et al. (1978) Immunochemistry *15*, 941; Lenkei and Ghetie (1977) J. Immunol. Methods *16*, 23 and Imai et al. (1979) Gastroenterology *76*, 242. The interaction of PHALB with Clq and the relationship of serologic reactivity with PHALB in sera from patients with and without liver disease have been reported by Milich et al. (1980) Gastroenterology *79*, 1116, and Milich et al. (1981) Gastroenterology *81*, 218.

A safe inexpensive vaccine is provided by employing polymerized human albumin in a physiologically acceptable carrier as a vaccine for hepatitis in the absence of other hepatitis virus derived immunogens. By polymerizing human albumin to provide at least a hexamer, an active immunogen is produced which upon injection produces an immunogenic response to PHALB, but not to monomeric albumin, affording the host protection from hepatitis.

Polymerized human serum albumin (PHALB) is prepared and employed in a physiologically acceptable carrier with an adjuvant as a vaccine for hepatitis virus. The polymerized albumin composition is desirably on the average at least a hexamer, preferably about an octamer and should generally be less than about a dodecamer, preferably less than about a decamer (6—12; 7—10), wherein 90 or greater weight percent is of the same degree of polymerization, more preferably having the average composition of an octamer. The polymerized human albumin can be formed in any conventional way, by aging, using heat or light, by chemical cross linking, for example, with aldehydes e.g. formaldehyde, dialdehydes, e.g. glutaraldehyde, or other physiologically acceptable tanning agents. Conditions can be chosen so as to optimize the formation of the desired degree of polymerization, followed by purification, if desired. Sedimentation or centrifugation may be employed for large scale separation by molecular weight, employing isopycnic banding, gradient density chromatography centrifugation, or molecular sieving by gel chromatography in large columns. The polymerized human albumin may then be chemically or thermally aggregated using physiologically acceptable multivalent cations or heat.

The manner of application may be varied widely. Any of the conventional methods for administration of a dead vaccine are applicable. These include oral application on a solid physiologically acceptable base or in a physiologically acceptable dispersion or parenterally, by injection. The dosage of the vaccine will depend on the route of administration and will vary according to the size of the host. Because the vaccine has few if any side effects, relatively large dosages may be used without injury to the host. Normally, the amount of the vaccine will be from about 1 µg to 20.0 mg per kilogram of host, more usually from about 5 µg to 2.0 mg given subcutaneously or intramuscularly after mixing with an appropriate carrier or an adjuvant to enhance immunization with the vaccine.

Various methods of achieving adjuvant effect for the vaccine includes use of agents such as aluminium hydroxide or phosphate (alum), commonly used as 0.05 to 0.1 percent solution in phosphate buffered saline, admixture with synthetic polymers of sugars (Carbopol®) used as 0.25 percent solution, aggregation of the protein in the vaccine by heat treatment with temperatures ranging between 70° to 101°C for 30 to 2 minute periods respectively, aggregation by reacting with pepsin treated (Fab) antibodies to albumin, mixture with bacterial cells such as *C. parvum* or endotoxins or lipopolysaccharide components of gram-negative bacteria, emulsion in physiologically acceptable oil vehicles such as mannide mono-oleate (Aracel A®) or emulsion with 20 percent solution of a perfluorocarbon (Fluosol-DA®) used as a blood substitute. More novel methods of adjuvanticity would include bacterial toxins against which the host has been preimmunized, for example by coupling 5 moles of the vaccine per mole of tetanus toxoid or diphtheria toxoid, the compound vaccine could elicit enhanced immune response to PHALB. The amount of the adjuvant will vary widely depending upon the nature of the adjuvant, generally varying from 0.1 to 100 times the weight of the immunogen, more usually from 1 to 10 times.

In many instances, it will be desirable to have multiple administrations of the vaccine, usually not exceeding six vaccinations, more usually not exceeding four vaccinations and preferably one or more, usually at least about three vaccinations. The vaccinations will normally be at from two to twelve week intervals, more usually from three to five week intervals. Periodic boosters at intervals of 1—5 years, usually three years, will be desirable to maintain protective levels of the antibodies. The course of the immunization may be followed by assays for antibodies for PHALB. The assays may be performed by labelling PHALB with conventional labels, such as radionuclides, enzymes, fluorescers, and the like. These techniques are well known and may be found in a wide variety of patents, such as U.S. Patent Nos. 3,791,932, 4,174,384, and 3,949,064, as illustrative of these types of assays. The types of assays may be divided between homogeneous, which do not involve a separation step, and heterogeneous, which do involve a separation step.

Radioimmunoassays are illustrative of the heterogeneous assays. A radioimmunoassay could be performed by binding PHALB to a surface, either a particle or the surface of a container, adding the serum sample to the bound PHALB, and allowing the mixture to incubate for sufficient time for any antibody to PHALB to react with the bound PHALB. One would then add radionuclide-labelled PHALB to the container, incubate for a period of time sufficient for the labelled PHALB to bind any antibody bound to the surface, wash, and then measure the radioactivity bound to the surface. Alternative protocols may also be employed.

In a homogeneous assay, PHALB could be substituted with fluorescer molecules, the labelled PHALB combined with the sample, followed by addition of antibodies to the fluorescer. Depending upon the choice of the fluorescer, binding of the antibody to the fluorescer could result in an increase or decrease in fluorescence. The binding of antibodies to the PHALB will inhibit binding of antibodies to the fluorescer, so that by measuring the fluorescence of the assay medium in comparison with an assay medium having known amounts of antibodies to PHALB, the presence of antibodies to PHALB in the sample could be determined. The particular manner in which the presence of the antibodies to the PHALB in a serum sample is determined is not a significant aspect of this invention.

The following examples are offered by way of illustration and not by way of limitation.

Experimental

To prepare cross-linked PHALB, 120 mg of crystalline human serum albumin (HSA) is dissolved in 3.6 ml of 0.1 M phosphate buffer (pH 6.8). Polymerization is accomplished by the addition of 0.4 ml of a 2.5% solution of glutaraldehyde solution in 0.1 M phosphate buffer (pH 6.8). After two hours at room temperature, the reaction mixture is dialyzed against $3 \times 500$ ml PBS, changed at 3, 6 and 18 hour intervals. The PHALB is then treated with 0.1% sodium borohydride to stabilize the polymer and neutralize unreacted aldehyde groups and then chromatographed through Sephacryl S-300 gel column in PBS. The first major peak is separated and determined to have an average molecular weight of 500,000 daltons.

For the performance of radioimmunoassay, HSA is prelabelled with $^{125}$I using the chloramine-T or the Hunter-Bolten reagent and then subjected to the above polymerization procedure to obtain $^{125}$I-PHALB probe. Polystyrene beads of 0.5 cm diameter are first coated with a 0.5 mg/ml solution of PHALB in PBS. The PHALB-coated beads are washed with PBS containing 0.2% Tween 20 and 0.5% BSA (pH 6.4) and air dried. Test sera are serially diluted with PBS and 2.5% BSA (pH 7.2) for titration and to each dilution a PHALB-coated bead is added, incubated for 2 hours at 37°C and then the beads are washed four times with deionized water. Then each bead is reacted with $^{125}$I-labelled PHALB probe, incubated for 4 hours at 37°C, washed $4\times$ and the radioactivity bound to the bead is counted in a gamma counter. The highest dilution of the serum sample giving counts of more than 2.1 times the negative control is considered positive and the end-point of titration. The negative control beads are coated with polymerized transferrin.

Several human sera from patients with acute hepatitis A, sera from convalescent hepatitis B and sera from chronic nonA/nonB hepatitis reveal the presence of antibodies to PHALB in the gamma globulin fraction. The serologic specificity of the antibodies is established by neutralization reactions using 4 units of limiting antibodies. Both 0.1 µg of PHALB and purified 3.0 µg of hepatitis virus envelope protein neutralize the antibodies, whereas up to 1000 µg of HSA and other plasma proteins fail to inhibit the human antibodies to PHALB. Thus, it is apparent that purified envelope protein of HBV and PHALB share the serologic determinant and indicate that antibodies to PHALB would provide an effective protection against infection by various hepatotropic viruses including the hepatitis B virus.

For preparation of the vaccine, 6.0 mg of PHALB is mixed with 20 mg of mannitol per ml of PBS and heated at 60°C for 11 hours, followed by heating at 101°C for 1—2 minutes. The heat-treated PHALB is mixed with equal volume of 0.1% aluminium hydroxide and tested for toxicity and pyrogenicity. The PHALB may then be used as a vaccine for immunization and formulated in the conventional ways. The mineral ion (aluminium) is required for induction of primary immune response, however, because it forms prolonged granuloma at the site of immunization, for the booster the heat-treated PHALB alone, without the adjuvanticity of alum, may be used for secondary immunization.

Effective immunization can be monitored by serologic tests of host serum for antibodies to PHALB. The primary immune response of IgM-type is distinguished from the IgC-type secondary immune response by treatment of the test serum with 0.1 M 2-mercaptoethanol. The secondary immune response to PHALB is long lasting and protective against infection with hepatotropic viruses.

In accordance with the subject invention, an inexpensive safe vaccine is provided which can be repeatedly administered to mammalian hosts to provide for a rapid immune response in the event of hepatitis infection by any of the hepatotrophic viruses. Human serum albumin can be readily obtained in pure form and then polymerized to physiological compositions. Thus, populations which cannot afford vaccines prepared by more expensive techniques can have protection from hepatitis.

**Claims**

1. A vaccine useful for vaccination for a mammalian host against hepatitis which comprises polymerized human serum albumin having from six to twelve units and being free of other proteins or genetic material derived from hepatotropic viruses, an immunogenic adjuvant and a physiologically acceptable carrier.

2. A vaccine according to claim 1, wherein said polymerized serum albumin is thermally or chemically aggregated.

3. A vaccine according to claim 1 or claim 2, wherein said polymerized serum albumin is on the average an octamer.

4. A process for preparing a vaccine useful for vaccination for a mammalian host against hepatitis, which comprises polymerizing human serum albumin to produce a polymerized serum albumin having from six to twelve monomer units per polymer unit and being free of other proteins or genetic material derived from hepatotropic viruses, and admixing the polymerized serum albumin with an immunogenic adjuvant and a physiologically acceptable carrier.

5. A process according to claim 4, wherein said polymerized serum albumin is thermally or chemically aggregated.

6. A process according to claim 4 or claim 5, wherein said polymerized serum albumin is on the average an octamer.

**Patentansprüche**

1. Ein Impfstoff, der nützlich für die Impfung eines Säugetier-Wirtes gegen Hepatitis ist, umfassend polymerisiertes menschliches Serumalbumin mit 6—12 Einheiten und frei von anderen Eiweißstoffen oder genetischem Material, das aus hepatotropen Viren abgeleitet ist, einen immunogenen Hilfsstoff und ein physiologisch verträgliches Trägermittel.

2. Ein Impfstoff nach Anspruch 1, worin das genannte polymerisierte Serumalbumin thermisch oder chemisch aggregiert ist.

3. Ein Impfstoff nach Anspruch 1 oder 2, worin das polymerisierte Serumalbumin durchschnittlich ein Octamer ist.

4. Verfahren zur Herstellung eines Impfstoffes, der nützlich für die Impfung eines Säugetier-Wirtes gegen Hepatitis ist, umfassend das Polymerisieren von menschlichen Serumalbumin unter Bildung eines polymerisierten Serumalbumins mit 6 bis 12 Monomereinheiten pro Polymereinheit, das frei ist von anderen Proteinen oder genetischem Material, das aus hepatotropen Viren abgeleitet ist, und Vermischung des polymerisierten Serumalbumins mit einem immunogenen Hilfsstoff und einem physiologisch verträglichen Träger.

5. Verfahren nach Anspruch 4, worin das polymerisierte Serumalbumin thermisch oder chemisch aggregiert wird.

6. Verfahren nach Anspruch 4 oder 5, bei dem ein polymerisiertes Serumalbumin gebildet wird, das durchschnittlich ein Octamer ist.

**Revendications**

1. Vaccin utile pour la vaccination d'un hôte mammifère contre l'hépatite qui comprend de l'albumine de sérum humain polymérisée ayant de six à douze unités et étant exempte d'autres protéines ou matière génétique dérivées des virus hépatotropes, un adjuvant immunogène et un véhicule physiologiquement acceptable.

2. Vaccin selon la revendication 1 où ladite albumine de sérum polymérisée est agrégée thermiquement ou chimiquement.

3. Vaccin selon la revendication 1 ou la revendication 2 où ladite albumine de sérum polymérisée est en moyenne un octamère.

4. Procédé pour la préparation d'un vaccin utile pour la vaccination d'un hôte mammifère contre l'hépatite, qui comprend la polymérisation d'albumine de sérum humain pour produire une albumine de sérum polymérisée ayant de six à douze unités monomères par unité de polymère et étant exempte d'autres protéines ou matière génétique dérivées de virus hépatotropes, et le mélange de l'albumine de sérum polymérisée avec un adjuvant immunogène et un véhicule physiologiquement acceptable.

5. Procédé selon la revendication 4, où ladite albumine de sérum polymérisée est agrégée thermiquement ou chimiquement.

6. Procédé selon la revendication 4 ou la revendication 5, où ladite albumine de sérum polymérisée est en moyenne un octamère.